# EUROPEAN PATENT APPLICATION

(11) **EP 2 840 077 A1**
(43) Date of publication of application: **25.02.2015**
(21) Application number: 13181064.0
(22) Date of filing: 20.08.2013
(51) Int. Cl.: C07C 253/30, C07C 255/07

(54) **Process for the manufacture of hydrogenated nitriles**

(71) Applicant: DSM IP Assets B.V., 6411 TH Heerlen (NL)
(72) Inventor: Beumer, Raphael, 79541 Lörrach (DE); Bonrath, Werner, 79115 Freiburg (DE); Lehmann, Hajo, 68220 Buschwiller (FR); Medlock, Jonathan Alan, 4310 Rheinfelden (CH)
(74) Representative: Steck, Melanie

(57) **Abstract**

The present invention relates to the manufacture and the use of specific organic compounds of formula (II) 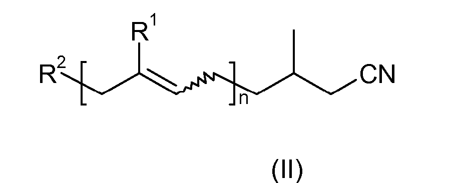 wherein n is either 1 or 2, and wherein R¹ is linear C₁₋₄ alkyl or branched C₃₋₄ alkyl, wherein R² is hydrogen or linear C₁₋₄ alkyl or branched C₃₋₄ alkyl, as aroma ingredients in flavors and fragrances. Furthermore the invention relates to new specific organic compounds and their synthesis, as well as to flavor and fragrance formulations comprising at least one of the specific organic compounds.

## Description

The present invention is directed a process for the manufacture of a compound of formula (II) 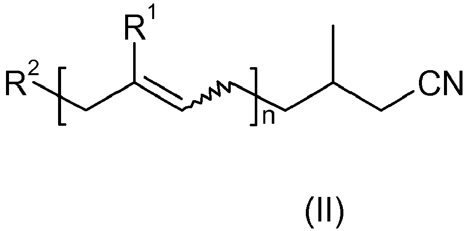 by reduction of a compound of formula I 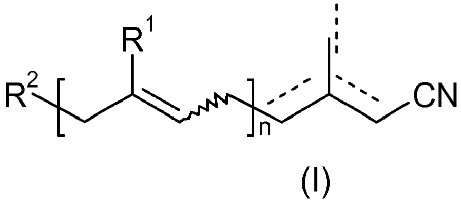 with hydrogen in the presence of a catalyst comprising nickel, wherein an additive is present, and
wherein n is either 1 or 2, preferably wherein n = 1;
wherein R¹ is linear C₁₋₄ alkyl or branched C₃₋₄ alkyl,
preferably wherein R¹ is methyl or ethyl, and
wherein R² is hydrogen or linear C₁₋₄ alkyl or branched C₃₋₄ alkyl,
preferably wherein R² is hydrogen or methyl, and
more preferably wherein either R¹ is methyl or R² is hydrogen.

Linear C₁₋₄ alkyl encompasses methyl, ethyl, n-propyl and n-butyl, whereas branched C₃₋₄ alkyl encompasses iso-propyl, iso-butyl and tert-butyl.

The reduction with hydrogen (the hydrogenation) of the compound of formula (I) is carried out in an organic solvent or a mixture of organic solvents or without using any solvent. One embodiment of the present invention is a solvent-free hydrogenation of the compound of formula (I). "Solvent-free" means without the usual amounts of such solvents. It is meant that no solvent is added to the starting materials, the compounds of formula (I).

But it is possible that the compounds of formula (I) may comprise traces of a solvent, which can originate from their production. But the amount of such solvent impurities is small, less than 10 weight-% and generally less than 2 weight-%.

### Solvent

If the hydrogenation is carried out in an organic solvent, the organic solvent is preferably selected from the group consisting of
0 aliphatic hydrocarbons (preferred: linear, branched or cyclic aliphatic hydrocarbons having 5 - 10 carbon atoms; more preferred: linear aliphatic hydrocarbons having 5 - 10 carbon atoms, i.e. n-pentane, n-hexane, n-heptane, n-octane, n-nonane and n-decane; even more preferred: linear aliphatic hydrocarbons having 6 - 8 carbon atoms, i.e. n-hexane, n-heptane, n-octane),
0 aromatic hydrocarbons (preferred: aromatic hydrocarbons having 5 - 10 carbon atoms),
0 chlorinated aliphatic hydrocarbons (preferred: chlorinated linear or branched aliphatic hydrocarbons; more preferred: chlorinated linear aliphatic hydrocarbons having 1 to 2 carbon atoms),
0 esters,
0 ethers and
0 alcohols, and any mixtures thereof;
more preferably the organic solvent is selected from the group consisting of aliphatic hydrocarbons, chlorinated aliphatic hydrocarbons and any mixtures thereof with the preferences as given above.

It is preferred that solvents are used which are liquid under normal conditions, which allows an easy handling, such as e.g. n-heptane (preferred) or dichloromethane.

The amount of the solvent is preferably chosen in such a way so that the solvent forms by weight 20 to 98% of the total solution to be hydrogenated, more preferably wherein the amount of the solvent is chosen in such a way so that the solvent forms by weight 50 to 95% of the total solution to be hydrogenated, even more preferably wherein the amount of the solvent is chosen in such a way so that the solvent forms by weight 70 to 90% of the total solution to be hydrogenated.

### Hydrogen

Usually the reduction with hydrogen (the hydrogenation) is carried out by using H₂-gas.
It is possible (and preferred) to use pure H₂-gas, but it would also be possible to use a gas mixture which comprises H₂.
Therefore the present invention relates to a process as described above wherein the hydrogenation is carried out by using (pure) H₂-gas.

### Reaction conditions

The hydrogenation is usually carried out under pressure, preferably at an absolute hydrogen pressure in the range of 1 to 20 bar, more preferably at an absolute hydrogen pressure in the range of 2 to 11 bar, even more preferably at a hydrogen pressure in the range of 4 to 9 bar, most preferably at an absolute hydrogen pressure of ca. 6 bar.

The hydrogenation is usually carried out in a vessel, which is suitable to enduring the pressure.

The hydrogenation is preferably carried out at a temperature in the range of 10 to 100°C, more preferably at a temperature in the range of 20 to 80°C, even more preferably at a temperature in the range of 40 to 60°C, most preferably at a temperature in the range of 45 to 55°C.

### Catalyst comprising nickel

The hydrogenation is carried out in the presence of a catalyst comprising nickel.
The catalyst comprising nickel used in the process according to the present invention is a heterogeneous catalyst.
Preferred catalysts are nickel-alloy type catalysts. Such catalysts are also known as "skeletal catalysts" or "sponge-metal catalysts".
Such catalysts are commercially available for example under the trade name Actimet^{®} from BASF (i.e. Actimet M) or under the product name B 111W, BLM 112 W, B 113 W, B 113 Z from Evonik or JM A4000, JM A40A9, JM A2000 from Johnson Matthey Catalysts or Acticat^{®} from CatAlloy (i.e. Acticat^{®}1000, Acticat^{®}1100, Acticat^{®}1200, Acticat^{®}1600).

Preferably the catalyst comprises an amount of nickel in the range of 75 to 95% by weight and an amount of aluminium in the range of 0 to 15% by weight, with the remainder made up by other metals such that the total weight sums to 100%. These other metals are e.g. iron, chromium and/or molybdenum.

In a further preferred embodiment of the present invention, the catalyst is promoted with iron or chromium or both, preferably in an amount of 0 to 10% by weight, based on the total weight of the catalyst.

The catalyst can be reused for further hydrogenation reactions and the catalyst can also be easily recycled.
Usually the catalyst can be used without further treatment. So it is possible to run the hydrogenation batch-wise or continuously.

Preferably the amount of the catalyst is in the range of 1 to 100 weight-%, more preferably the amount of the catalyst is in the range of 10 to 80 weight-%, even more preferably the amount of the catalyst is in the range of 30 to 60 weight-%, based on the amount of the compound of formula (I).

### Additive

Suitable additives are selected from the group of additives containing a nitrogen or a sulfur atom, preferably from the group of additives containing a sulfur atom.

Preferably the amount of the additive is in the range of 0.01 to 100 weight-%, more preferably the amount of the additive is in the range of 0.1 to 10 weight-%, even more preferably the amount of the additive is in the range of 0.2 to 2 weight-%, based on the amount of the catalyst.

### Additives containing a sulfur atom

Suitable additives containing a sulfur atom are aromatic disulfides, aliphatic disulfides, aromatic sulfides, heteroaromatic sulfides, aliphatic sulfides, aromatic thiols, heteroaromatic thiols, aliphatic thiols, each containing a total number of 2-15 carbon atoms and a total number of 1 or 2 sulfur atoms, respectively.

Preferred additives containing a sulfur atom are aliphatic symmetrical disulfides, aromatic symmetrical disulfides such as diphenyldisulfide, aliphatic symmetrical sulfides, aromatic symmetrical sulfides such as diphenylsulfide, and heteroaromatic sulfides such as thiophene.

Examples of aliphatic symmetrical disulfides are disulfides R-S-S-R with R being straight chain C₂₋₆ alkyl. Examples of aliphatic symmetrical sulfides are sulfides R-S-R with R being straight chain C₂₋₆ alkyl, i.e. R being ethyl, n-propyl, n-butyl, n-pentyl and n-hexyl.

The most preferred additives containing a sulfur atom are diethyl disulfide, diphenyl disulfide, diethyl sulfide, diphenyl sulfide, diethylendisulfide, thiophene and 2,2'-(ethylenedithio)diethanol, whereby diethyldisulfide (H₃C-CH₂-S-S-CH₂-CH₃) is especially preferred.

### Additives containing a nitrogen atom

Suitable additives containing a nitrogen atom are aromatic primary amines, aromatic secondary amines, aromatic tertiary amines; heteroaromatic primary amines, heteroaromatic secondary amines, heteroaromatic tertiary amines; aliphatic primary amines, aliphatic secondary amines, aliphatic tertiary amines; amidines and guanidines; each containing a total number of 3 to 10 carbon atoms and 1 to 3 nitrogen atoms.

Examples of amidines R-C(=NR³)-NR¹R² are ones wherein R is C₁₋₆ alkyl and R¹, R² and R³ are independently from each other hydrogen or straight chain C₁₋₆ alkyl. Additionally the alkyl groups may be linked together to form a ring.

Examples of guanidines R¹R²N(C=NR)NR³R⁴ are ones wherein R-R⁴ are independently from each other hydrogen or straight chain C₁₋₄ alkyl, preferably wherein R-R⁴ are independently from each other hydrogen, methyl or ethyl, with the proviso that at least one of R-R⁴ is not hydrogen; more preferably wherein all R-R4 are methyl.

Preferably the additive containing a nitrogen atom is selected from the groups of C4-C10 aromatic heterocycles containing 1-2 nitrogen atoms and C4-C10 aliphatic heterocycles containing 1-2 nitrogen atoms, C6-C10 bicyclic aliphatic amidines, C2-C8 aliphatic amines and C2-C6 aliphatic diamines.

Examples of C4-C10 aromatic heterocycles containing 1-2 nitrogen atoms are pyrrol, pyridine and quinoline. Examples of C4-C10 aliphatic heterocycles containing 1-2 nitrogen atoms are piperidine, pyrrolidine and quinuclidine. An example of a C6-C10 bicyclic aliphatic amidine is 1,8-diaza-bicyclo[5.4.0]undec-7-ene (= "DBU").

The most preferred additives containing a nitrogen atom are pyridine, quinoline, quinuclidine, 1,8-diaza-bicyclo[5.4.0]undec-7-ene, ethylene diamine, 1,4-diaminobutane and triethylamine.

### Description of the drawings

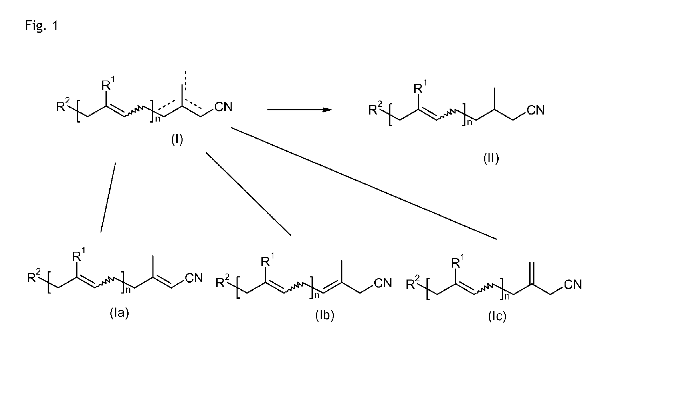

Fig. 1 illustrates the reaction of compounds of formula (I) to compounds of formula (II) according to the present invention showing the C=C double bond isomers as concrete compounds Ia, Ib and Ic;

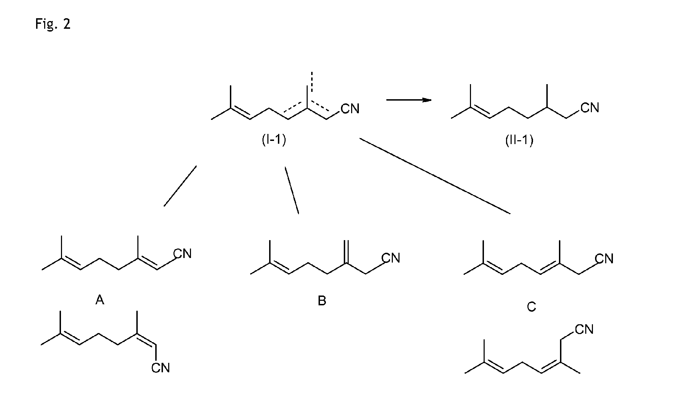

Fig. 2 illustrates the hydrogenation of a mixture of geranyl nitrile isomers (compound of formula I-1), i.e. of a mixture of (*E*/*Z*)-3,7-dimethyl-2,6-octadiene nitrile (compound A), 7-methyl-3-methylene-6-octene nitrile (compound B) and (*E*/*Z*)-3,7-dimethyl-3,6-octadiene nitrile (compound C) to citronellyl nitrile (compound of formula II-1);

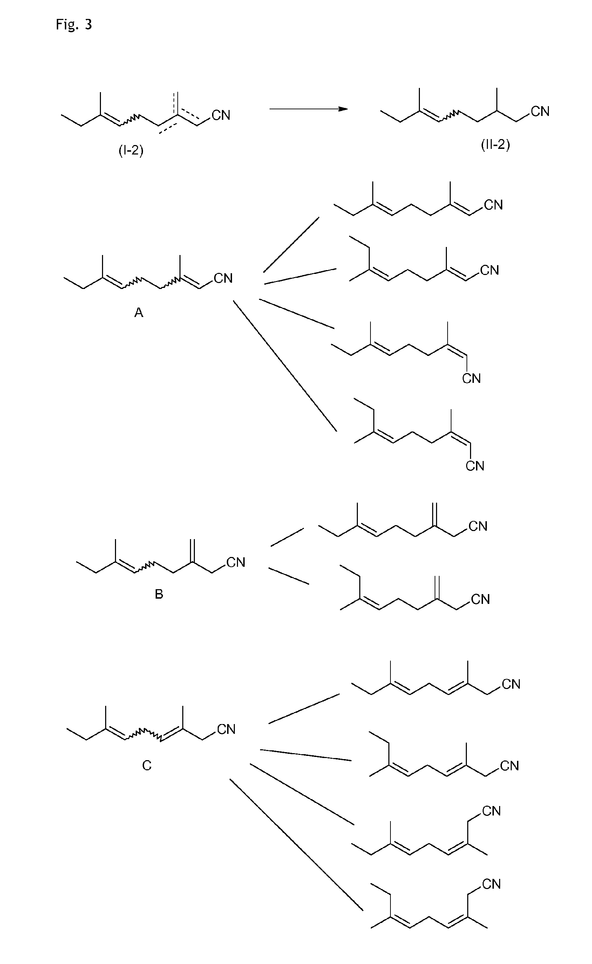

Fig. 3 illustrates the hydrogenation of methyl limonitrile (compound of formula I-2); i.e. a mixture of 3,7-dimethyl-2,6-nonadiene nitrile (compound A), 7-methyl-3-methylene-6-nonene nitrile (compound B) and 3,7-dimethyl-3,6-nonadiene nitrile (compound C) to (*E*/*Z*)-3,7-dimethyl-6-nonennitrile (compound of formula II-2).

Besides a mixture of geranyl nitrile isomers of formula I-1 it is also possible to hydrogenate any other mixture of geranyl nitrile ((E)-3,7-dimethyl-2,6-octadiene nitrile) and neryl nitrile ((Z)-3,7-dimethyl-2,6-octadiene nitrile) or the single compounds according to the process of the present invention to obtain citronellyl nitrile. Since geranyl nitrile is toxic there is an increasing demand of substituting geranyl nitrile by another fragrance such as a compound of formula (II), which does not have the toxicological disadvantages of geranyl nitrile.

### Novel compounds

Since the compounds of formula (II) - with the proviso that R² is not hydrogen if R¹ = methyl and n = 1 - are novel, the present invention is also directed to them. Thus, a further embodiment of the present invention is a compound of formula (II) 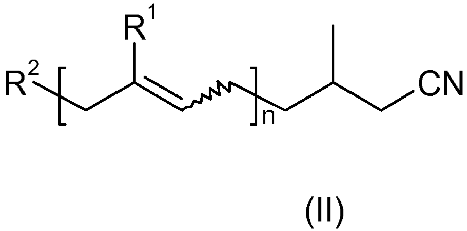 wherein n is either 1 or 2, preferably wherein n = 1;
wherein R¹ is linear C₁₋₄ alkyl or branched C₃₋₄ alkyl,
preferably wherein R¹ is methyl or ethyl, and
wherein R² is hydrogen or linear C₁₋₄ alkyl or branched C₃₋₄ alkyl,
preferably wherein R² is hydrogen or methyl,
with the proviso that R² is not hydrogen if R¹ = methyl and n = 1.

### Novel use

Furthermore, the invention also relates to the use of a compound of formula (II) with the proviso that R² is not hydrogen if R¹ = methyl and n = 1, especially of a compound of formula II-2, as aroma ingredient.

In the flavor and fragrance industry there is always a need and demand for compounds that enhance, modify, improve or otherwise positively influence an odor note and therefore give perfumers or other persons the ability to create new fragrances for perfumes, colognes, personal care products, household products or any other products, which comprise aroma ingredients, i.e. well-defined substances with characteristic aroma. Flavors and fragrances are compositions made of several aroma ingredients.

Surprisingly it was found that the compounds of formula (II), with the proviso that R² is not hydrogen if R¹ = methyl and n = 1, especially the compound of formula II-2, are very useful in flavor and fragrance compositions.
Therefore the present invention is related to the use of a compound of formula (II) with the proviso that R² is not hydrogen if R¹ = methyl and n = 1, especially of a compound of formula II-2, in flavor and/or fragrance applications.
The compounds of formula (II) with the proviso that R² is not hydrogen if R¹ = methyl and n = 1, especially the compound of formula II-2, may be used as such or in combination with other compounds of formula (II) or other compounds which are known as aroma ingredients.

Such other compounds which are known as aroma ingredients include all known odorant molecules selected from the extensive range of natural products and synthetic molecules currently available, such as essential oils, alcohols, aldehydes and ketones, ethers and acetals, esters and lactones, macrocycles and heterocycles, and/or mixed with one or more ingredients or excipients conventionally used in conjunction with odorants in flavor or fragrance formulations, for example, carrier materials, and other auxiliary agents commonly used in the art.

The aroma ingredients of the present invention, i.e. the compounds of formula II, are used in a flavor and fragrance formulation.
Such a flavor and fragrance formulation comprises other ingredients.
The flavor and fragrance formulation according to the present invention can be in any form. Usually it is in a solid, gel-like or liquid (or a combination thereof) form. It can also be in an encapsulated form (i.e. a liquid formulation encapsulated by a suitable matrix material).

Therefore the present invention also relates to flavor and fragrance formulations comprising
(i) at least one compound of formula (II) with the proviso that R² is not hydrogen if R¹ = methyl and n = 1, especially the compound of formula II-2.

When a compound of formula (II) is used in a flavor and fragrance formulation, then the amount thereof is in the range of 0.0001 - 10 weight-% (wt-%), related to the total weight of the flavor and fragrance formulation. Preferably is an amount in the range of 0.01 - 5 wt-%, based on the total weight of the flavor and fragrance formulation.

Therefore the present invention relates to liquid flavor and fragrance formulations comprising
(i) 0.0001 - 10 wt-% (preferably 0.01 - 5 wt-%), related to the total weight of the flavor and fragrance formulation, of at least one compound of formula (II) with the proviso that R² is not hydrogen if R¹ = methyl and n = 1, especially of a compound of formula II-2.

The flavor and fragrance formulations according to the present invention can comprise further ingredients (= auxiliary compounds), such as any further perfuming compounds, solvents, adjuvants, thickeners, surface active agents, pigments, extenders, rheology modifiers, dyestuffs, antioxidants, fillers and the like.

Many flavor and fragrance formulations are in a liquid form (like a perfume, cologne, etc.). Therefore, for such liquid formulation a (diluent) solvent is present. Such common diluents are i.e. dipropyleneglycol, isopropyl myristate, triethylcitrate and alcohols (such as ethanol).

Further examples of fine perfumery are Eau de perfume, Eau de Toilette, Eau de Cologne and Splash Cologne. Fine perfumery products are commonly based on an alcoholic solution as diluent. However fine perfumery products using an oil or wax as diluent are also included within the meaning of this invention. The compounds can be employed in widely varying amounts, depending upon the specific application and on the nature and quantity of other odourant ingredients.

When used in a (fine) perfume, the amount of the compound of formula (II) with the proviso that R² is not hydrogen if R¹ = methyl and n = 1, especially of a compound of formula II-2, is usually between 0.01 - 10 wt-%, based on the total weight of the (fine) perfume.
However, these values and ranges are given only by way of example, since the experienced perfumer may also achieve effects or may create novel accords with lower or higher concentrations.

Furthermore the present invention relates to liquid flavor and fragrance formulations comprising
(i) at least one compound of formula (II) with the proviso that R² is not hydrogen if R¹ = methyl and n = 1, especially the compound of formula II-2, and
(ii) at least one diluent chosen from the group consisting of dipropyleneglycol, isopropylmyristate, triethylcitrate and alcohols (such as ethanol), and optionally
(iii) at least one auxiliary compound selected from the group consisting of perfuming compounds, solvents, adjuvants, thickeners, surface active agents, pigments, extenders, rheology modifiers, dyestuffs, antioxidants and fillers.

Furthermore the present invention relates to solid flavor and fragrance formulations comprising
(i) at least one compound of formula (II) with the proviso that R² is not hydrogen if R¹ = methyl and n = 1, especially the compound of formula II-2, and
(ii) at least one auxiliary compound selected from the group consisting of perfuming compounds, solvents, adjuvants, thickeners, surface active agents, pigments, extenders, rheology modifiers, dyestuffs, antioxidants and fillers.

The compounds of formula (II) with the proviso that R² is not hydrogen if R¹ = methyl and n = 1, especially the compound of formula II-2, may be used in a broad range of flavor and fragrance formulations, e.g. in any field of fine and functional perfumery, such as perfumes, air care products, household products, laundry products, body care products and cosmetics.

The compounds as described hereinabove may be employed in a flavor and fragrance formulation simply by directly mixing at least one compound of formula (II), a mixture thereof, or a fragrance composition with the other ingredients used in the final product, or they may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the other ingredients used in the final product.

Thus, the invention additionally provides a method of manufacturing a flavor and fragrance formulation, comprising the incorporation of a compound of formula (II) with the proviso that R² is not hydrogen if R¹ = methyl and n = 1, especially of the compound of formula II-2, as a fragrance ingredient, either by directly admixing the compound to the other ingredients used in the final product or by admixing a fragrance composition comprising a compound of formula (I), which may then be mixed with the other ingredients used in the final product, using conventional techniques and methods.

Through the addition of an olfactory acceptable amount of a compound of the present invention as hereinabove described, or a mixture thereof, the odor notes of a consumer product base will be improved, enhanced or modified.

Thus, the invention furthermore provides a method for improving, enhancing or modifying a flavor or fragrance formulation as well as a consumer product (= final product) base by means of the addition thereto of an olfactory acceptable amount of a compound of formula (II) with the proviso that R² is not hydrogen if R¹ = methyl and n = 1, especially of the compound of formula II-2, or a mixture thereof.

In the context of the present invention the olfactory effective amount is to be understood as the amount of the at least one compound of formula (II) with the proviso that R² is not hydrogen if R¹ = methyl and n = 1, especially of the compound of formula II-2, in a flavor and fragrance formulation which will contribute to its particular olfactory characteristics, but the olfactory effect of the flavor and fragrance formulation will be the sum of the effects of each of the perfumes or fragrance ingredients. Thus the compounds of the invention can be used to alter the aroma characteristics of the flavor and fragrance formulation, or by modifying the olfactory reaction contributed by another ingredient in the composition. The amount will vary depending on many factors including other ingredients, their relative amounts and the effect that is desired.

As used herein, "consumer product (= final product)" means a composition for use as a consumer product to fulfill specific actions, such as cleaning, softening, and caring or the like. Examples of such products include fine perfumery, e.g. perfume and eau de toilette; fabric care, household products and personal care products such as laundry care detergents, rinse conditioner, personal cleansing composition, detergent for dishwasher, surface cleaner; laundry products, e.g. softener, bleach, detergent; body care products, e.g. shampoo, shower gel; air care products and cosmetics, e.g. deodorant, vanishing creme. This list of products is given by way of illustration and is not to be regarded as being in any way limiting.

The invention is now further illustrated in the following non-limiting examples.

### Examples

3,7-dimethyl-2,6-octadiene nitrile is purchased from Sigma Aldrich; purity 97%, as a mix of 2E- and 2Z-isomers with a ratio of approximately 1 : 1.

### Examples 1-5: Hydrogenation of (E,Z)-3,7-dimethyl-2,6-octadiene nitrile

### Small scale experiments

### Example 1

The mixture of geranyl nitrile and neryl nitrile, the solvent, catalyst and additive (if required) are added in the amounts given in **table 1** to a glass reactor. The reactor is sealed and is purged with argon 5 times (by pressurising to 5 bar followed by release of the pressure) and 3 times with hydrogen (pressurise to 3 bar then release). The reaction mixture is heated to the desired temperature, pressurised to the desired hydrogen pressure and stirred at 1000 rpm. At the end of the reaction the pressure is released and the reactor purged 3 times with argon. After filtration to remove the catalyst, the reaction mixture is analysed by GC to determine conversion and selectivity.

### Comparison examples A-D

These experiments are performed in the same manner as example 1 with the solvent, the catalyst, the additive and the reaction conditions as given in **table 1.**

### Larger scale experiments

### Example 2

This experiment is carried out in an analogous manner as example 1. Details about the solvent, the catalyst, the additive and the reaction conditions are given in **table 2.**

### Examples 3-5

The mixture of geranyl nitrile and neryl nitrile, the solvent, catalyst and additive are added in the amounts given in **table 2** to a 500 ml stainless steel reactor. The reactor is sealed and is purged with nitrogen 3 times (by pressurising to 5 bar followed by release of the pressure) and 3 times with hydrogen (pressurise to 5 bar then release). The reaction mixture is heated to the desired temperature, pressurised to the desired hydrogen pressure and stirred at 1000 rpm. At the end of the reaction the pressure is released and the reactor purged once with nitrogen. After filtration to remove the catalyst, the reaction mixture is analysed by GC to determine conversion and selectivity.

The results (conversion and selectivity) of the experiments 1-5 (according to the invention) and the comparison examples A-D are listed in **table 1** and **2.**

**Table 1: Hydrogenation of a 1:1 mixture of 3,7-dimethyl-2(Z),6-octadiene nitrile and 3,7-dimethyl-2(E),6-octadiene nitrile Example 1: according to the invention; examples A-D: comparison examples; T = Temperature**

| **Example** | **Amount of Starting Material** | **Solvent (amount)** | **Catalyst (amount)** | **Additive (amount)** | **Time (hours)** | **T (°C)** | **Pressure H₂ (bar abso-lute)** | **Conversion (%)** | **Selectivity (%)** |
|---|---|---|---|---|---|---|---|---|---|
| **1** | 200 mg | Heptane (2.0 g) | Nickel Alloy Johnson Matthey A4000 (160 mg) | Diethyldisulfide (6 mg) | 25 | 50 | 6 | 98 | 91 |
| **A** | 270 mg | Heptane (2.7 g) | 5% Pd/C Evonik E 101 N/D (5 mg) | Triethylamine (69 mg) | 6 | 50 | 6 | 20 | 19 |
| **B** | 360 mg | Heptane (3.4 g) | 5% Pd/C Evonik E 101 N/D (5 mg) | DBU (2 mg) | 26 | 80 | 11 | 3 | 2 |
| **C** | 200 mg | Dichloromethane (2.2 g) | 5% Pd/CaCO₃ Evonik | None | 22 | 25 | 6 | 22 | 8 |
| **D** | 1.0 g | Heptane (10 g) | 5% Pd/Al₂O₃ Evonik | None | 24 | 25 | 11 | 100 | 35 |

**Table 2: Hydrogenation of a 1:1 mixture of 3,7-dimethyl-2(Z),6-octadiene nitrile and 3,7-dimethyl-2(E),6-octadiene nitrile Examples 2-5: according to the invention, T = temperature**

| **Example** | **Starting Material** | **Solvent (amount)** | **Catalyst (amount)** | **Additive (amount)** | **Time (hours)** | **T (°C)** | **Pressure H₂ (bar absolute)** | **Conversion (%)** | **Selectivity (%)** |
|---|---|---|---|---|---|---|---|---|---|
| **2** | 2.0 g | Heptane (20 g) | Nickel Alloy Johnson Matthey A4000 (1.0 g) | Diethyldisulfide (50 mg) | 65 | 50 | 6 | 100 | 91 |
| **3** | 20.0 g | Heptane (200 g) | Nickel Alloy Johnson Matthey A4000 (10 g) | Diethyldisulfide (450 mg) | 49 | 50 | 6 | 100 | 95 |
| **4** | 20.0 g | Heptane (200 g) | Nickel Alloy Johnson Matthey A4000 (9 g) | Diethyldisulfide (360 mg) | 40 | 50 | 6 | 94 | 87 |
| **5** | 10.0 g | Heptane (200 g) | Nickel Alloy Johnson Matthey A4000 (6 g) | Diethyldisulfide (180 mg) | 21 | 50 | 6 | 97 | 88 |

## Claims

1. A process for the manufacture of a compound of formula II 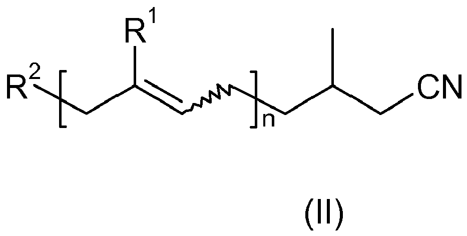 by reduction of a compound of formula I 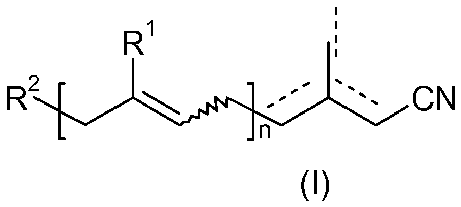 with hydrogen in the presence of a catalyst comprising nickel, wherein an additive is present, and
wherein n is either 1 or 2, preferably wherein n is 1;
wherein R¹ is linear C₁₋₄ alkyl or branched C₃₋₄ alkyl,
preferably wherein R¹ is methyl or ethyl, and
wherein R² is hydrogen or linear C₁₋₄ alkyl or branched C₃₋₄ alkyl,
preferably wherein R² is hydrogen or methyl, and
more preferably wherein either R¹ is methyl or R² is hydrogen.

2. The process according to claim 1, wherein the reduction is carried out in an organic solvent, preferably selected from the group consisting of aliphatic hydrocarbons, aromatic hydrocarbons, chlorinated aliphatic hydrocarbons, esters, ethers and alcohols, and any mixtures thereof, more preferably selected from the group consisting of aliphatic hydrocarbons, chlorinated aliphatic hydrocarbons and any mixtures thereof, most preferably selected from the group consisting of n-heptane and dichloromethane; and/or wherein the amount of the solvent is chosen in such a way so that the solvent forms by weight 20 to 98% of the total solution to be hydrogenated, preferably wherein the amount of the solvent is chosen in such a way so that the solvent forms by weight 50 to 95% of the total solution to be hydrogenated, more preferably wherein the amount of the solvent is chosen in such a way so that the solvent forms by weight 70 to 90% of the total solution to be hydrogenated.

3. The process according to any of the preceding claims, wherein the additive is selected from the group of additives containing a nitrogen or a sulfur atom, preferably wherein the additive is selected from the group of additives containing a sulfur atom, more preferably wherein the additive containing a sulfur atom is selected from the group consisting of diethyl disulfide, diphenyl disulfide, diethyl sulfide, diphenyl sulfide, diethylendisulfide, thiophene and 2,2'-(ethylenedithio)diethanol; and/or wherein the amount of the additive is in the range of 0.01 to 100 weight-%, preferably wherein the amount of the additive is in the range of 0.1 to 10 weight-%, more preferably wherein the amount of the additive is in the range of 0.2 to 2 weight-%, based on the amount of the catalyst.

4. The process according to any of the preceding claims, wherein the reduction with hydrogen is carried out at a temperature in the range of 10 to 100°C, preferably at a temperature in the range of 20 to 80°C, more preferably at a temperature in the range of 40 to 60°C, even more preferably at a temperature in the range of 45 to 55°C; and/or at an absolute hydrogen pressure in the range of 1 to 20 bar, preferably at an absolute hydrogen pressure in the range of 2 to 11 bar, more preferably at a hydrogen pressure in the range of 4 to 9 bar, most preferably at an absolute hydrogen pressure of ca. 6 bar.

5. The process according to any of the preceding claims, wherein the catalyst is of a nickel-alloy type, preferably wherein the catalyst comprises an amount of nickel in the range of 75 to 95% by weight and an amount of aluminium in the range of 0-15% by weight with the remainder made up by other metals such that the total weight sums to 100%.

6. The process according to claim 5, wherein the catalyst is promoted with iron or chromium or both, preferably in an amount of 0 to 10% by weight, based on the total weight of the catalyst.

7. The process according to any of the preceding claims, wherein the amount of catalyst is in the range of 1 to 100 weight-%, preferably wherein the amount of catalyst is in the range of 10 to 80 weight-%, more preferably wherein the amount of catalyst is in the range of 30 to 60 weight-%, based on the amount of the compound of formula (I).

8. Use of a compound of formula (II) with the proviso that R² is not hydrogen if R¹ = methyl and n = 1 as aroma ingredient.

9. Use of a compound of formula (II) with the proviso that R² is not hydrogen if R¹ = methyl and n = 1 in flavour and/or fragrance applications.

10. A flavor or fragrance formulation comprising
(i) at least one compound of formula (II) with the proviso that R² is not hydrogen if R¹ = methyl and n = 1.

11. The flavor and fragrance formulation according to claim 10 comprising 0.0001 - 10 wt-% of at least one compound of formula (II), related to the total weight of the flavor and fragrance formulation.

12. The flavor and fragrance formulation according to claim 10 and/or 11, wherein the flavor and fragrance formulation is solid, gel-like or liquid.

13. The flavor and fragrance formulation according to any one or more of claims 10 - 12, wherein the flavor and fragrance formulation is a perfume, air care product, household product, laundry product, body care product or cosmetic product.

14. A method of improving, enhancing or modifying a flavor or fragrance formulation by means of addition thereto an olfactory acceptable amount of at least one compound of formula (II) with the proviso that R² is not hydrogen if R¹ = methyl and n = 1.

15. A compound of formula (II) 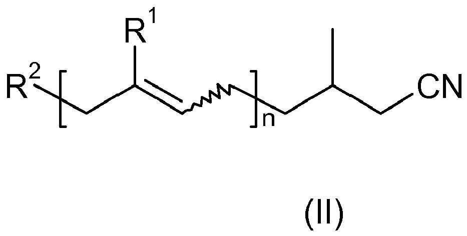 wherein n is either 1 or 2, preferably wherein n = 1; and
wherein R¹ is linear C₁₋₄ alkyl or branched C₃₋₄ alkyl,
preferably wherein R¹ is methyl or ethyl, and
wherein R² is hydrogen or linear C₁₋₄ alkyl or branched C₃₋₄ alkyl,
preferably wherein R² is hydrogen or methyl,
with the proviso that R² is not hydrogen if R¹ = methyl and n = 1.
